Europäisches Patentamt

European Patent Office

Office européen des brevets

(19)

(11) **EP 0 616 792 B1**

(12) **EUROPÄISCHE PATENTSCHRIFT**

(45) Veröffentlichungstag und Bekanntmachung des Hinweises auf die Patenterteilung:
**03.06.1998 Patentblatt 1998/23**

(51) Int. Cl.$^6$: **A61B 5/087**, G01F 1/50, G01F 25/00

(21) Anmeldenummer: **94103226.0**

(22) Anmeldetag: **03.03.1994**

(54) **Referenzimpedanz für die Kalibration der Druck- und Flowmesseinrichtung eines Gerätes zur oszillometrischen Messung des Atemwegwiderstands**

Oscillometric determination of the airway impedance, by use of a standard impedance provided for the calibration of a pressure and flow measuring device

Détermination oscillométrique de l'impédance des voies respiratoires à l'aide d'une impédance étalon destinée au calibrage d'un dispositif de mesure de pression et de débit

(84) Benannte Vertragsstaaten:
**AT BE DE ES FR GB IT NL**

(30) Priorität: **05.08.1993 DE 4326374**
**08.03.1993 DE 9303369 U**

(43) Veröffentlichungstag der Anmeldung:
**28.09.1994 Patentblatt 1994/39**

(73) Patentinhaber: **Erich Jaeger GmbH**
**D-97204 Hoechberg (DE)**

(72) Erfinder:
• **Smith, Hans-Jürgen, Dipl.-Ing.**
  **D-97082 Würzburg (DE)**
• **Vogel, Johannes, Dr.**
  **D-13125 Berlin-Karow (DE)**
• **Eichler, Rüdiger, Dipl.-Ing.**
  **D-97225 Zellingen (DE)**
• **Arnold, Johannes, Dipl.-Ing.**
  **D-97082 Würzburg (DE)**

(74) Vertreter:
**Heusler, Wolfgang, Dipl.-Ing. et al**
**Dr. Dieter von Bezold**
**Dipl.-Ing. Peter Schütz**
**Dipl.-Ing. Wolfgang Heusler**
**Brienner Strasse 52**
**80333 München (DE)**

(56) Entgegenhaltungen:
EP-A- 0 161 003          EP-A- 0 328 415
DE-A- 3 529 367          US-A- 5 060 655

• Eur Respir Rev, 1991, 1, Rev 3, 158-162, Cauberghs et al: "Calibration Procedure of the forced oscillation technique".

EP 0 616 792 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die Erfindung betrifft die Verwendung einer Referenzimpedanz für die dynamische Kalibration der Druck- und Flowmeßeinrichtung eines Lungenfunktionsprüfgerätes zur oszillometrischen Messung des Atemwegwiderstandes (thorakopulmonale Impedanz) gemäß dem Oberbegriff des Anspruchs 1 sowie ein die Referenzimpedanz verwendendes Meßgerät.

Bei der oszillometrischen Bestimmung des Atemwegwiderstandes werden am Mund des Patienten Druckschwingungen appliziert und üblicherweise mit einem Druckwandler und einem Pneumotachographen die sich ergebenden Werte für Druck und Flow gemessen. Hieraus kann man dann den Atemwegwiderstand als Quotient aus Druck und Flow ermitteln. Bei der Impulsoszillometrie (IOS) ist das applizierte Meßsignal ein mit einem Lautsprecher durch Anlegen eines elektrischen Rechteckimpulses erzeugter kurzzeitiger Druckimpuls, der in seinem Spektrum die für die Untersuchung benötigten Frequenzkomponenten von 0 bis 50 Hz enthält.

Bei derartigen Messungen müssen frequenzbezogene Verfälschungen der Flow- und Drucksignale aufgrund geometrischer, pneumatischer und elektrischer Übertragungsfunktionen der Meßeinrichtung berücksichtigt und eliminiert werden. Zum Kalibrieren der Druck- und Flowmeßeinrichtung in ihrem dynamischen Verhalten, d.h. im Frequenzgang, wird deshalb bei der Oszillometrie, die ja auch die Frequenzebene zur Berechnung von Lungenfunktionsparametern heranzieht, an Stelle des Patienten eine Referenzimpedanz bekannter Größe angeschlossen. Mit Hilfe der in ihren Werten bekannten Referenzimpedanz läßt sich der Einfluß der Meßeinrichtung auf Ergebnisse der Messung numerisch erfassen. So kann ein Korrekturalgorithmus bestimmt werden, der diesen Einfluß eliminiert.

Für den routinemäßigen Einsatz oszillometrischer Verfahren ist die Kontrolle der dynamischen Kalibration im Hinblick auf die Vergleichbarkeit gemessener Parameter von eminenter Bedeutung.

Bisher wurden für diesen Zweck Referenzimpedanzen verwendet, die neben der rein resistiven Komponente erhebliche kapazitive und/oder induktive Widerstandskomponenten besitzen (die kapazitive Komponente entspricht hier bekanntlich der adiabatischen Komplianz oder Kompressibilität des Gases, während die induktive Komponente der Massenträgheit des Gases entspricht). Ein typisches Beispiel hierfür ist ein zylindrisches Rohr, in das metallische Drahtsiebe eingesetzt sind (Eur Respir Rev, 1991, 1, Rev 3, 158-162). Derartige Impedanzen zeigen insbesondere bei relativ großen Flowamplituden, wie sie bei der Impulsoszillometrie auftreten, kein lineares Verhalten. Eine andere bekannte Impedanz, die aus einem Bündel einer Vielzahl dünner Glasröhrchen besteht (Eur Respir Rev, 1991, 1, Rev 3, 163-166) erweitert zwar den Bereich des linearen Strömungsverhaltens, hat aber eine größere induktive Komponente. Die reaktiven Komponenten der bekannten Referenzimpedanzen sind nur für bestimmte Flowwerte definiert, sie sind also flowabhängig.

Aus der EP-A-0 161 003 ist es an sich bekannt, als Mundstück für ein Lungenfunktionsprüfgerät wie namentlich ein Spirometer eine Impedanz zu verwenden, die ein resistiv wirkendes Widerstandselement mit quer zur Strömungsrichtung verlaufender Anströmfläche und ein mit dem Widerstandselement über eine Strömungsanlaufstrecke verbundenes Rohrstück zum Anschluß an die Meßeinrichtung hat, wobei die Anströmfläche wesentlich größer ist als die Querschnittsfläche des Rohrstücks an der Stelle des Anschlusses an die Meßeinrichtung und die Anlaufstrecke sich bis zum Umfang der Anströmfläche stetig erweitert. Das bekannte Mundstück zeichnet sich durch einen bekannten und in Abhängigkeit vom Flow ungefähr linearen Differenzdruck aus.

Der Erfindung liegt die Aufgabe zugrunde, die Verwendung einer insbesondere für die Impulsoszillometrie geeigneten Referenzimpedanz anzugeben, die ein einfaches Kalibrieren ermöglicht und dabei einen genau reproduzierbaren und weitgehend flowunabhängigen Widerstandswert hat.

Diese Aufgabe wird durch die Verwendung der im Anspruch 1 gekennzeichneten Referenzimpedanz gelöst.

Durch die Erfindung ist es erstmals möglich, das Verhalten der Meßeinrichtung lastabhängig auch bei größeren Flowamplituden und bei sehr unterschiedlichen Geräten zuverlässig zu messen und in einer entsprechenden Systemkorrektur zu berücksichtigen.

Besonders vorteilhaft ist die verwendete Referenzimpedanz, weil sie erfindungsgemäß so ausgestaltet sein kann, daß die nicht resistiven Komponenten der Impedanz vernachlässigbar klein sind. Eine gegebenenfalls noch vorhandene geringe Reaktanz ist ebenfalls genau definierbar und flowunabhängig. Hierdurch wird die Kalibration erheblich vereinfacht. Der rein resistive Siebwiderstand ist in Betrag und Phase im interessierenden Frequenzbereich bis 50 Hz frequenzlinear.

Besonders bei dem oben erwähnten routinemäßigen Einsatz oszillometrischer Verfahren zeigen sich die Vorteile der verwendeten Referenzimpedanz, die keine komplexen Impedanzcharakteristiken erzeugt, sondern einen definierten frequenzunabhängigen Resistanzverlauf und eine Reaktanz im Nullbereich.

An einem Ausführungsbeispiel wird die Erfindung näher erläutert. In der Zeichnung zeigen:

Fig. 1     eine schematische Darstellung einer bevorzugten Ausführungsform der Referenzimpedanz;

Fig. 2     den Aufbau eines dreilagigen Siebwiderstandes der Referenzimpedanz, dessen mittlere Sieblage ein ausgestanztes Loch enthält, über dessen Größe der absolute Siebwiderstandswert hochgenau abgleichbar ist;

Fig. 3 den Meßkopf eines IOS-Prüfgerätes, an den die hier beschriebene Referenzimpedanz angeschlossen ist;

Fig. 4 Kurven des Druck-Flow-Verlaufes bei Referenzimpedanzen mit unterschiedlichen Widerstandswerten; und

Fig. 5 typische Werte der Resistanz und Reaktanz einer Referenzimpedanz gemäß Fig. 1 und 2.

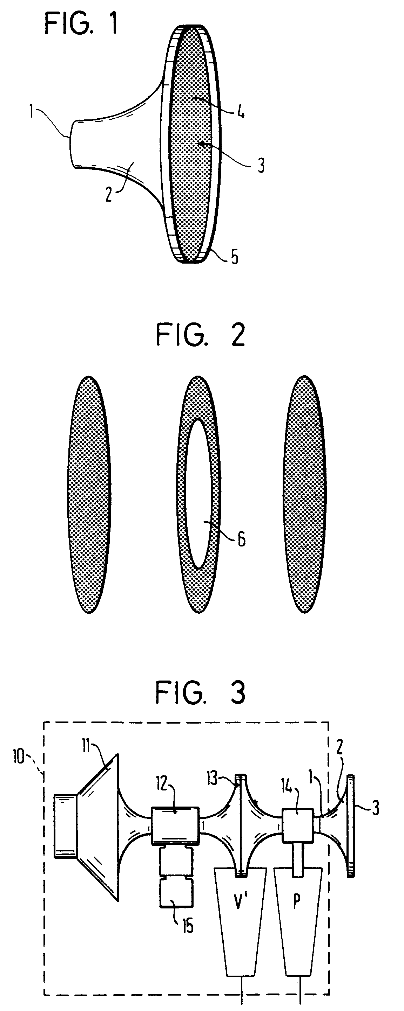

Wie in Fig. 1 erkennbar ist, besteht die Referenzimpedanz im wesentlichen aus einem flachen, scheibenförmigen Siebwiderstandselement 3, das am freien Ende einer nach außen geschlossenen Strömungsanlaufstrecke 2 mit kreisförmigem Querschnitt montiert ist, die am anderen Ende in ein zum Anschluß an die Meßeinrichtung dienendes, beispielsweise allgemein zylindrisches oder konisches Anschlußrohrstück 1 übergeht und sich von diesem bis zum Umfang der senkrecht zur Strömungsrichtung liegenden (in Fig. 1 nicht sichtbaren) kreisrunden Anströmfläche annähernd parabolisch oder trompetenförmig erweitert. Das Strömungsprofil erweitert sich vom Anschlußrohrstück 1 mit einem Innendurchmesser von z.B. 26 mm auf einen Siebwiderstandsdurchmesser von z.B. 55 mm. Dabei beträgt der Abstand zwischen dem Anschlußkonus, d.h. dem Ende des Anschlußrohrstücks 1, und dem Sieb etwa 40 mm. Das aus dem Rohrstück 1 und der Anlaufstrecke 2 gebildete Formteil besteht aus starrem Kunststoffmaterial. Insbesondere die Anlaufstrecke soll starre Wände haben.

Die zur Anlaufstrecke, d.h. zum Strömungskanal entgegengesetzte Seite 4 des Siebwiderstandselements 3 liegt zur Umgebung offen und frei (im Gegensatz zu bekannten Pneumotachographen, die den Druckabfall an einem Siebwiderstand zwischen zwei einander ähnlichen Rohrstücken bestimmen).

Die bei 5 angedeutete, aus einem Kunststoffbügel und/oder einer Metallfassung bestehende Verschlußkonstruktion zur auswechselbaren Halterung des Siebwiderstandselements 3 bedeckt nur den Rahmen des Metallsiebes und engt den aktiven Siebquerschnitt nicht ein.

Das Siebwiderstandselement 3 besteht vorzugsweise aus drei aufeinanderliegenden Sieblagen gleicher Größe, die aus demselben beispielsweise gewebten, insbesondere metallischen Sieb- oder Netzmaterial hergestellt sein können. Vorzugsweise findet ein Edelstahlgewebe mit einer Drahtstärke von 30 μm und einer Maschenweite von 33 μm Anwendung (die Werkstoffbezeichnung lautet 1.4301). Während die beiden außenliegenden Sieblagen die in Fig. 2 dargestellte, ununterbrochene Oberfläche haben, ist aus der mittleren Sieblage eine im Vergleich mit den Siebmaschen große zentrale kreisrunde Öffnung 6 (beispielsweise mit einem Durchmesser von ungefähr 3 cm) ausgestanzt oder auf andere Weise ausgenommen. Über die Größe der ausgestanzten Fläche läßt sich der absolute Widerstandswert hochgenau einstellen.

Weiterhin bewirkt das ausgestanzte Loch in Verbindung mit den beiden äußeren Sieblagen und dem Kunststofformteil einen linearen Frequenzgang ohne Resonanzstellen im Bereich von 0 bis 50 Hz.

Fig. 3 zeigt den Meßkopf 10 eines nach dem bekannten Prinzip der Impulsoszillometrie (IOS) arbeitenden Lungenfunktionsprüfgerätes zum Messen des Atemwegwiderstandes des Menschen, für das die beschriebene Referenzimpedanz erfindungsgemäß verwendet wird. Der Meßkopf enthält den Lautsprecher 11 zum Erzeugen der Testimpulse. Die Modulation des Atemstromes durch das Testsignal findet im Y-Verbindungsstück 12 statt. Der Patient atmet über z.B. aus Siebwiderständen mit definierten Werten bestehende Abschlußwiderstände 15 zur Umgebungsluft. Dem Atemstrom überlagert werden die im Y-Verbindungsstück 12 durch den Lautsprecheranschluß zugeführten Testsignale. Der vom Testsignalfluß überlagerte Atemstrom (V') wird in üblicher Weise mit einem Pneumotachograph 13 am Munde des Patienten gemessen. Zusätzlich befindet sich direkt zwischen einem Mundstück und dem Pneumotachograph ein Drucksensor 14, der den Munddruck (P) feststellt. Die Meßergebnisse stehen als elektrische Signale zur Verfügung. Der Druck-Flow-Quotient ist die zu bestimmende thorakopulmonale Impedanz des Patienten.

Zum Kalibrieren des Meßkopfes wird dagegen am Ausgang des Drucksensors 14 die Referenzimpedanz gemäß Fig. 1 mit ihrem dem erwähnten Mundstück ähnlichen Anschlußkonus 1 angeschlossen.

Vorzugsweise ist das rein resistiv wirkende Siebwiderstandselement 3 am freien Ende der Strömungsanlaufstrecke 2 auswechselbar in einer von Hand lösbaren Schnellverschlußkonstruktion montiert; die schon erwähnte Verschlußkonstruktion 5 kann beispielsweise als Bajonettverschluß ausgebildet sein. Die leichte Austauschbarkeit der Siebwiderstände und ihre technologische Güte ermöglichen die Bereitstellung von Referenzimpedanzen, welche die gesamte Breite des physiologischen Impedanzspektrums des Menschen überdecken und damit eine noch genauere Überprüfung des Systemverhaltens erlauben.

Andererseits kann dieselbe Referenzimpedanz zum Justieren verschiedener Lungenfunktionsgeräte verwendet werden, so daß beispielsweise die Geräte verschiedener Labors verglichen und standardisiert werden können.

Wie schon erwähnt wurde, zeichnet sich die beschriebene Referenzimpedanz durch weitgehende Flowunabhängigkeit aus, d.h. man kann die Referenzimpedanz auch bei relativ großen Strömungsamplituden einsetzen. Strömungstechnisch stellt der zur Ankopplung an das System notwendige, vom Rohrstück 1 gebildete Anschlußkonus eine Lochblende dar, die ein quadratisches flowabhängiges Widerstandsverhalten hat. Diese Komponente, hier mit R* bezeichnet, kann grundsätzlich nicht eliminiert werden. Durch geeignete Wahl des Siebwiderstands läßt sich das Verhältnis zum nichtlinearen Teil aber beliebig zu Gunsten des Siebwiderstands vergrößern. Für die Impulsoszillometrie veranschaulicht die nachfolgende Tabelle, daß selbst ohne Berücksichtigung der quadratischen Eingangskomponente der Fehler bei Siebwiderständen größer als 2 hPa/l/s in vertretbaren Grenzen gehalten werden kann:

| Siebwiderstand $R_z$ hPa/l/s | Peakflow l/s | $R^*/R_z$ % |
|---|---|---|
| 2 | 1,2 | 1,3 |
| 1 | 1,7 | 3,6 |
| 0,36 | 2,2 | 12 |

Die vollständige Ersatzschaltung der Referenzimpedanz besteht demzufolge aus der Reihenschaltung von nichtlinearer Komponente R* und dem Siebwiderstand $R_z$. Die nichtlineare Komponente der Referenzimpedanz am Anschlußkonus berechnet sich nach der allgemeinen Formel

$$R^* = k \times V',$$

wobei der konstante Faktor k je nach Anschlußkonus zu ermitteln ist. Beispielsweise wurde für einen Anschlußkonus mit einem Innendurchmesser von 26 mm ein k von 2,56 hPa/(l/s)$^2$ bestimmt. Mathematisch ist es mit Hilfe der angegebenen Formel möglich, das Verhalten der Referenzimpedanz über einen Flowbereich bis 18 l/s zu linearisieren.

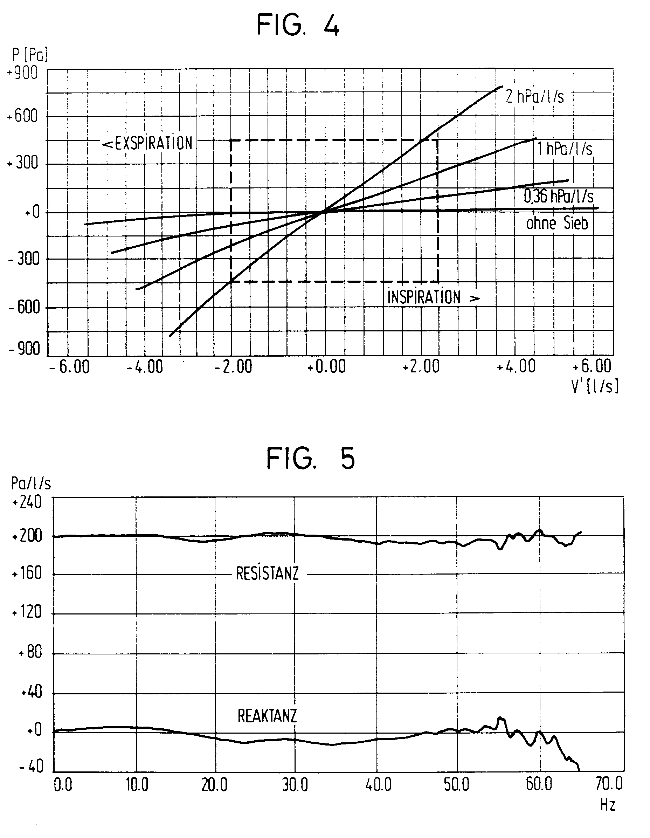

In Fig. 4 sind Druck-Flow-Verläufe dargestellt, die für verschiedene Referenzimpedanzen aufgezeichnet wurden, ohne Korrektur der nichtlinearen Widerstandskomponente. Flowamplituden, wie sie bei der Impulsoszillometrie auftreten, sind durch die gestrichelte Einrahmung hervorgehoben. Mindestens im Bereich dieser Einrahmung ist der Druck-Flow-Quotient hinreichend konstant, das Widerstandsverhalten demzufolge linear.

Neben der Flowunabhängigkeit wirkt die beschriebene Referenzimpedanz aufgrund zweckmäßiger Form der Strömungsanlaufstrecke in Verbindung mit dem Siebwiderstand im wesentlichen rein resistiv. Das in der Referenzimpedanz eingeschlossene Luftvolumen erzeugt eine für die Messung zu vernachlässigende Komplianz (Nachgiebigkeit), so daß diese Komponente für praktische Zwecke als Null definiert werden kann. Ähnliches gilt für die Induktanz.

Der typische Verlauf von Resistanz und Reaktanz einer bei praktischen Versuchen verwendeten Referenzimpedanz gemäß Fig. 1 von 200 Pa/l/s in Abhängigkeit von der Frequenz ist in Fig. 5 gezeigt. Dabei stellen die obere Kurve die gemessene Resistanz und der untere Verlauf die gemessene Reaktanz dar.

**Patentansprüche**

1. Verwendung einer Referenzimpedanz für die dynamische Kalibration der Druck- und Flowmeßeinrichtung eines Lungenfunktionsprüfgerätes zur oszillometrischen Messung des Atemwegwiderstandes des Menschen und/oder zum Korrigieren der mit dem Gerät gewonnenen Meßergebnisse, wobei die Referenzimpdanz ein resistiv wirkendes Widerstandselement (3) mit quer zur Strömungsrichtung verlaufender Anströmfläche, insbesondere einen kreisrunden Siebwiderstand, und ein mit dem Widerstandsselement (3) über eine Strömungsanlaufstrecke (2) verbundenes Rohrstück (1) zum Anschluß an die Meßeinrichtung (10) hat, und wobei die entgegengesetzt zu der Strömungsanlaufstrecke liegende Fläche (4) des Widerstandselements (3) zur Umgebung offen liegt, **dadurch gekennzeichnet,** daß eine Referenzimpedanz verwendet wird, bei der die Anströmfläche des resistiven Widerstandselements (3) wesentlich größer ist als die Querschnittsfläche des Rohrstücks (1) an der Stelle des Anschlusses an die Meßeinrichtung (10) und die Anlaufstrecke (2) sich bis zum Umfang der Anströmfläche stetig erweitert.

2. Verwendung einer Referenzimpedanz nach Anspruch 1, **dadurch gekennkennzeichnet,** daß die Anlaufstrecke (2) sich von einem zylindrischen oder konischen Rohrstück (1) parabolisch oder trompetenförmig zum Umfang der Anströmfläche des Widerstandselements (3) erweitert.

3. Verwendung einer Referenzimpedanz nach Anspruch 1 oder 2, **dadurch gekennzeichnet,** daß der Durchmesser der Anströmfläche des Widerstandselements (3) größer ist als die in Strömungsrichtung gemessene Länge des sich erweiternden Teils der Anlaufstrecke (2).

4. Verwendung einer Referenzimpedanz nach einem der Ansprüche 1 bis 3, **dadurch gekennzeichnet,** daß das Widerstandselement (3) mindestens drei aufeinanderliegende Sieblagen enthält und aus der mittleren Sieblage eine zentrale Öffnung (6) ausgenommen ist.

5. Verwendung einer Referenzimpedanz nach einem der vorangehenden Ansprüche, **dadurch gekennzeichnet,** daß das Widerstandselement (3) aus einem Metallgewebe mit einer Maschenweite von ungefähr 30 bis 40 μm gebildet ist.

6. Verwendung einer Referenzimpedanz nach einem der vorangehenden Ansprüche, **dadurch gekennzeichnet**, daß das resistive Widerstandselement (3) am freien Ende der Anlaufstrecke (2) auswechselbar in einer von Hand lösbaren Schnellverschlußkonstruktion (5) montiert ist.

7. Verwendung einer Referenzimpedanz nach einem der vorangehenden Ansprüche, **dadurch gekennzeichnet**, daß eine Mehrzahl auswechselbarer Widerstandselemente (3) mit unterschiedlichen Widerstandswerten verfügbar ist, die den für die Messung relevanten Teil des physiologischen Spektrums der Atemwegimpedanzen des Menschen abdecken.

8. Gerät zur oszillometrischen, insbesondere impulsoszillometrischen, Messung des Atemwegwiderstands mit einer einen Druckschwingungs- oder Druckimpulserzeuger (11), einen Flowmeßwandler (13) und einen Druckmeßwandler (14) enthaltenden Meßeinrichtung (10), an die ein Mundstück für den Patienten anschließbar ist, und mit einer anstelle des Mundstücks an die Meßeinrichtung (10) angeschlossenen Referenzimpedanz zum Kalibrieren des Gerätes und/oder zum Korrigieren der mit dem Gerät gewonnenen Meßergebnisse, die ein resistiv wirkendes Widerstandselement (3) mit quer zur Strömungsrichtung verlaufender Anströmfläche, insbesondere einen kreisrunden Siebwiderstand, und ein mit dem Widerstandselement (3) über eine Strömungsanlaufstrecke (2) verbundenes Rohrstück (1) zum Anschluß an die Meßeinrichtung (10) hat, wobei die entgegengesetzt zu der Strömungsanlaufstrecke liegende Fläche (4) des Widerstandselements (3) zur Umgebung offen liegt, **dadurch gekennzeichnet**, daß die Anströmfläche des resitiven Widerstandselements (3) wesentlich größer ist als die Querschnittsfläche des Rohrstücks (1) an der Stelle des Anschlusses an die Meßeinrichtung (10) und die Anlaufstrecke (2) sich bis zum Umfang der Anströmfläche stetig erweitert.

## Claims

1. Use of a reference impedance for dynamic calibration of the pressure and flow-measuring equipment of a lung-testing device for oscillometric measurement of human respiratory impedance and/or for correcting the measurement results obtained with the device, the reference impedance comprising a resistive resistance element (3) with an oncoming-flow surface extending transversely to the direction of flow, particularly a circular screen resistor, and

a pipe (1) connected to the resistance element (3) via a flow-starting section (2), for connection to the measuring equipment (10), and the surface (4) of the resistance element (3) opposite to the flow-starting section being open to atmosphere, characterized by using a reference inpedance in which the oncoming-flow surface area of the resistive resistance element (3) is considerably greater than the cross-sectional area of the pipe (1) at the place where it is connected to the measuring equipment (10), and the starting section (2) widens up continuously to the periphery of the oncoming-flow surface.

2. Use of a reference impedance according to claim 1, characterized in that the starting section (2) widens in parabolic or trumpet shape from a cylindrical or conical pipe (1) to the periphery of the oncoming-flow surface of the resistance element (3).

3. Use of a reference impedance according to claim 1 or 2, characterized in that the diameter of the oncoming-flow surface of the resistance element (3) is greater than the length, measured in the flow direction, of the widening part of the starting section (2).

4. Use of a reference impedance according to any of claims 1 to 3, characterized in that the resistance element (3) contains at least three superposed screen layers, and a central opening (6) is formed in the middle screen layer.

5. Use of a reference impedance according to any of the preceding claims, characterized in that the resistance element (3) comprises a metal fabric having a mesh width of about 30 to 40 μm.

6. Use of a reference impedance according to any of the preceding claims, characterized in that the resistive resistance element (3) is interchangeably fitted to the free end fo the starting section (2) in a manually releasable snap-fastener construction (5).

7. Use of a reference impedance according to any of the preceding claims, characterized in that a plurality of interchangeable resistance elements (3) with different resistance values are available which cover that part of the physiological spectrum of human respiratory impedance which is relevant to the measurement.

8. Device for oscillometric, particularly impulse-oscillometric measurement of the respiratory impedance, comprising measuring equipment (10) including a pressure oscillation or pressure pulse generator (11), a flow transducer (13) and a pressure transducer (14), a mouthpiece for the patient being connectable to said measuring equipment, and further comprising a reference impedance for calibration of the device and/or for correcting the measurement results obtained with the device, the reference impedance being connected to the measuring equipment (10) instead of the mouthpiece and having a resistive resistance element (3) with an oncoming-flow surface extending transversely to the direction of flow, particularly a circular screen resistor, and a pipe (1) connected to the resistance element (3) via a flow-starting section (2), for connection to the measuring equipment (10), the surface (4) of the resistance element (3) opposite to the flow-starting section being open to atmosphere, characterized in that the oncoming-flow surface area of the resistive resistance element (3) is considerably greater than the cross-sectional area of the pipe (1) at the place where it is connected to the measuring equipment (10), and the starting section (2) widens up to continuously to the periphery of the oncoming-flow surface.

**Revendications**

1.   Utilisation d'une impédance de référence pour l'étalonnage dynamique du dispositif de mesure de la pression et du débit d'un appareil d'examen de l'état de fonctionnement des poumons, pour la mesure par oscillométrie de la résistance du circuit respiratoire de l'être humain et/ou pour la correction des résultats de la mesure obtenus au moyen de l'appareil, l'impédance de référence comportant un élément de résistance (3) agissant d'une manière résistive, à surface d'arrivée du courant s'étendant perpendiculairement à la direction du courant, constituée en particulier par une résistance à tamis circulaire, et une pièce tubulaire (1) reliée à l'élément de résistance (3) par l'intermédiaire d'un tronçon d'arrivée du courant (2), pour son raccordement à l'appareil de mesure (10), la surface (4) de l'élément de résistance (3) qui est située à l'opposé du tronçon d'arrivée du courant (2), étant ouverte sur l'environnement, caractérisée en ce qu'on utilise une impédance de référence dans laquelle la surface d'arrivée du courant de l'élément de résistance (3) est sensiblement plus grande que la section transversale de la pièce tubulaire à l'endroit de son raccordement au dispositif de mesure (10) et le tronçon d'arrivée du courant (2) s'étend jusqu'à la périphérie de la surface d'arrivée du courant en s'évasant régulièrement.

2.   Utilisation d'une impédance de référence suivant la revendication 1 caractérisée en ce que le tronçon d'arrivée du courant (2) va en s'évasant sous une forme parabolique ou de trompette, à partir d'une pièce tubulaire (1) cylindrique ou conique, jusqu'à la surface d'arrivée du courant de l'élément de résistance (3).

3.   Utilisation d'une impédance de référence suivant l'une quelconque des revendications 1 ou 2 caractérisée en ce que le diamètre de la surface d'arrivée du courant de l'élément de résistance (3) est plus grand que la longueur, mesurée dans la direction du courant, de la partie évasée du tronçon d'arrivée du courant (2).

4.   Utilisation d'une impédance de référence suivant l'une quelconque des revendications 1 à 3 caractérisée en ce que l'élément de résistance (3) comporte au moins trois couches de tamis superposées et une ouverture centrale (6) est formée dans la couche de tamis intermédiaire.

5.   Utilisation d'une impédance de référence suivant l'une des revendications précédentes caractérisée en ce que l'élément de résistance (3) est constitué d'une toile métallique à ouverture de maille allant d'environ 30 à 40 micromètres.

6.   Utilisation d'une impédance de référence suivant l'une des revendications précédentes caractérisée en ce que l'élément de résistance (3) résistif est monté amovible, à l'extrémité libre du tronçon d'arrivée du courant (2), dans un raccord rapide détachable manuellement.

7.   Utilisation d'une impédance de référence suivant l'une des revendications précédentes caractérisée en ce qu'une pluralité d'éléments de résistance (3) interchangeables, ayant des valeurs de leurs résistances différentes, sont disponibles, ces éléments couvrant la partie du spectre physiologique des impédances de circuits respiratoires des êtres humains qui est concernée par la mesure.

8.   Appareil de mesure par oscillométrie, en particulier par oscillométrie à impulsions, de la résistance du circuit respiratoire au moyen d'un dispositif de mesure (10) comportant un générateur d'oscillations ou d'impulsions de pression (11), un débitmètre (13) et un manomètre (14), dispositif auquel peut être raccordée une pièce de bouche pour les patients, et une impédance de référence raccordée au dispositif de mesure (10) à la place de la pièce de bouche, en vue de l'étalonnage de l'appareil et/ou de la correction des résultats de mesure obtenus au moyen de l'appareil, cette impédance de référence comprenant un élément de résistance (3) agissant d'une manière résistive, à surface d'arrivée du courant s'étendant perpendiculairement à la direction du courant, constituée en particulier par une résistance à tamis circulaire, et une pièce tubulaire (1) reliée à l'élément de résistance (3) par l'intermédiaire d'un tronçon d'arrivée du courant (2), pour son raccordement à l'appareil de mesure (10), la surface (4) de l'élément de résistance (3) qui est située à l'opposé du tronçon d'arrivée du courant (2), étant ouverte sur l'environnement, caractérisée en ce que la surface d'arrivée du courant de l'élément de résistance (3) est sensiblement plus grande que la section transversale de la pièce tubulaire à l'endroit de son raccordement au dispositif de mesure (10) et le tronçon d'arrivée du courant (2) s'étend jusqu'à la périphérie de la surface d'arrivée du courant en s'évasant régulièrement.

FIG. 1

FIG. 2

FIG. 3

# FIG. 4

# FIG. 5